# EUROPÄISCHE PATENTANMELDUNG

(11) **EP 3 366 660 A1**
(43) Veröffentlichungstag der Anmeldung: **29.08.2018**
(21) Anmeldenummer: 17157933.7
(22) Anmeldetag: 24.02.2017
(51) Int. Cl.: C07C 5/48, C07C 11/04, C07C 51/215, C07C 51/44, C07C 51/48, C07C 53/08

(54) **VERFAHREN UND ANLAGE ZUR HERSTELLUNG VON OLEFINEN**

(71) Anmelder: Linde Aktiengesellschaft, 80331 München (DE)
(72) Erfinder: Höfel, Torben, 81543 München (DE)
(74) Vertreter: Frommberger, Moritz

(57) **Zusammenfassung**

Es wird ein Verfahren (100) zur Herstellung von Ethylen, bei dem Ethan in einem Reaktionseinsatz durch oxidative Dehydrierung (1) unter Anwesenheit von Sauerstoff unter Erhalt eines gasförmigen ersten Komponentengemischs, das zumindest Ethan, Ethylen, Essigsäure und Wasser enthält, teilweise katalytisch umgesetzt wird, vorgeschlagen. Dabei ist vorgesehen, dass zumindest ein Teil des gasförmigen ersten Komponentengemischs unter Erhalt eines flüssigen zweiten Komponentengemischs, das Wasser und Essigsäure enthält, einer Wäsche mit einer Waschflüssigkeit unterworfen wird, dass ein erster Anteil des zweiten Komponentengemischs zur Bildung der Waschflüssigkeit verwendet wird, dass ein zweiter Anteil des zweiten Komponentengemischs unter Erhalt eines flüssigen dritten Komponentengemischs, das zumindest ein organisches Lösungsmittel und Essigsäure enthält, einer Lösungsmittelextraktion unterworfen wird, und dass zumindest ein Teil des flüssigen dritten Komponentengemischs erwärmt und unter Erhalt einer überwiegend oder ausschließlich Essigsäure enthaltenden Flüssigkeit einer Destillation unterworfen wird. Die Erwärmung des dritten Komponentengemischs oder dessen der Destillation unterworfenen Teils wird zumindest teilweise im Wärmetausch mit dem ersten Komponentengemisch und/oder mit dem ersten und/oder mit dem zweiten Anteil des zweiten Komponentengemischs durchgeführt. Eine entsprechende Anlage ist ebenfalls Gegenstand der vorliegenden Erfindung.

## Beschreibung

Die Erfindung betrifft ein Verfahren zur Herstellung von Olefinen und eine entsprechende Anlage gemäß den Oberbegriffen der unabhängigen Patentansprüche.

### Stand der Technik

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den Olefinen gleicher Kohlenstoffzahl und zu Wasser umgesetzt.

Die ODH kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen keine thermodynamische Gleichgewichtslimitierung. Die ODH kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine insitu-Regenerierung ermöglicht. Schließlich werden im Gegensatz zum Steamcracken geringere Mengen an wertlosen Nebenprodukten wie Koks gebildet.

Zu weiteren Details bezüglich der ODH sei auf einschlägige Fachliteratur, beispielsweise Ivars, F. und López Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, verwiesen.

Bei der ODH werden insbesondere bei Verwendung von MoVNbTeOx-Katalysatoren unter industriell relevanten Reaktionsbedingungen signifikante Mengen der jeweiligen Carbonsäuren der eingesetzten Paraffine als Nebenprodukte gebildet. Für einen wirtschaftlichen Anlagenbetrieb ist eine entsprechende Koppelproduktion von Olefinen und der entsprechenden Carbonsäuren bei Verwendung des beschriebenen Katalysatortyps in der Regel zweckmäßig. Dies gilt insbesondere für die ODH von Ethan (sogenannte ODH-E), bei der gleichzeitig Essigsäure gebildet wird. Die Olefine und die Carbonsäuren müssen, wenn diese separat als Produkte bereitgestellt werden sollen, voneinander getrennt werden.

Zudem werden bei der ODH nennenswerte Mengen an u.a. Kohlenmonoxid und Kohlendioxid als Nebenprodukte gebildet, die zusammen mit Wasser, Restsauerstoff und Restethan ebenfalls in einem in der ODH gebildeten Gasgemisch enthalten sind und von den jeweiligen Hauptprodukten, also den Olefinen und den entsprechenden Carbonsäuren, abgetrennt werden müssen.

Die vorliegende Erfindung stellt sich die Aufgabe, eine entsprechende Trennung zu verbessern und insbesondere effizienter auszugestalten.

### Offenbarung der Erfindung

Vor diesem Hintergrund schlägt die vorliegende Erfindung ein Verfahren zur Herstellung von Olefinen und eine entsprechende Anlage mit den Merkmalen der unabhängigen Patentansprüche vor. Ausgestaltungen sind jeweils Gegenstand der abhängigen Patentansprüche sowie der nachfolgenden Beschreibung.

Stoffströme, Gasgemische usw. können im hier verwendeten Sprachgebrauch reich oder arm an einer oder mehreren Komponenten sein, wobei die Angabe "reich" für einen Gehalt von wenigstens 99%, 99,5%, 99,9% oder 99,99% und die Angabe "arm" für einen Gehalt von höchstens 1%, 0,5%, 0,1% oder 0,01% auf molarer, Gewichts- oder Volumenbasis stehen kann. Sind mehrere Komponenten angegeben, bezieht sich die Angabe "reich" oder "arm" auf die Summe aller Komponenten. Ist hier beispielsweise von "Sauerstoff" oder "Ethan" die Rede, kann es sich um ein Reingas, aber auch um ein an der jeweiligen Komponente reiches Gemisch handeln.

Stoffströme, Gasgemische usw. können im hier verwendeten Sprachgebrauch außerdem "angereichert" oder "abgereichert" an einer oder mehreren Komponenten sein, wobei sich diese Begriffe auf einen Gehalt in einem Ausgangsgemisch beziehen. Sie sind "angereichert", wenn sie zumindest den 1,5-fachen, 2-fachen, 5-fachen, 10-fachen, 100-fachen oder 1.000-fachen Gehalt, "abgereichert", wenn sie höchstens den 0,75-fachen, 0,5-fachen, 0,1-fachen, 0,01-fachen oder 0,001-fachen Gehalt einer oder mehrerer Komponenten, bezogen auf das Ausgangsgemisch, enthalten.

Nachfolgend werden zur Charakterisierung von Drücken und Temperaturen die Begriffe "Druckniveau" und "Temperaturniveau" verwendet, wodurch zum Ausdruck gebracht werden soll, dass Drücke und Temperaturen nicht in Form exakter Druck- bzw. Temperaturwerte vorliegen werden müssen. Ein Druckniveau oder Temperaturniveau kann beispielsweise ± 1%, 5%, 10%, 20% oder 50% um einen Mittelwert liegen. Mehrere Druck- und Temperaturniveaus können disjunkte oder überlappende Bereiche darstellen. Dasselbe Druck- bzw. Temperaturniveau kann beispielsweise auch dann noch vorliegen, wenn sich aufgrund von Leitungsverlusten oder Abkühlung Drücke und Temperaturen reduziert haben. Bei hier in bar angegebenen Druckniveaus handelt es sich um Absolutdrücke.

Bei einer "Destillationskolonne" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, eingespeistes Stoffgemisch durch Destillation bzw. Rektifikation zumindest teilweise aufzutrennen, also aus dem Stoffgemisch jeweils Reinstoffe oder zumindest Stoffgemische mit anderer Zusammensetzung zu erzeugen. Typischerweise sind Destillationskolonnen als zylindrische Metallbehälter ausgebildet, die mit Einbauten, beispielsweise Trennböden oder geordneten oder ungeordneten Packungen, ausgerüstet sind. Eine Destillationskolonne weist einen Sumpfverdampfer auf. Hierbei handelt es sich um eine Einrichtung mit einem Wärmetauscher, der beheizt wird und dafür eingerichtet ist, eine im Sumpf der Rektifikationskolonne anfallende flüssige Fraktion, auch als Sumpfflüssigkeit bezeichnet, zu erwärmen. Mittels eines Sumpfverdampfers wird kontinuierlich ein Teil des Sumpfprodukts verdampft und gasförmig in dem Trennbereich zurückgespeist.

Im hier verwendeten Sprachgebrauch wird hingegen unter einer "Waschkolonne" (engl. Scrubber) eine Trenneinheit verstanden, die dafür eingerichtet ist, ein gasförmig eingespeistes Gasgemisch einer als Waschflüssigkeit bzw. Absorptionsflüssigkeit bezeichneten Flüssigkeit entgegenzuschicken und hierdurch Komponenten des Gasgemischs aus diesem in die Waschflüssigkeit zu überführen. Bei den aus dem Gasgemisch in die Waschflüssigkeit überführten Komponenten kann es sich um feste, flüssige oder gasförmige Stoffe handeln, im vorliegenden Fall insbesondere um Wasser und Essigsäure, die in dem Gasgemisch in gasförmige oder fein verteilter flüssiger Form vorliegen. Auch eine Waschkolonne kann Einbauten aufweisen, die vergleichbar mit jenen einer Destillationskolonne ausgebildet sein können und bereitgestellt sind, um eine möglichst große Austauschfläche zwischen dem Gasgemisch und der Waschflüssigkeit herzustellen. Eine Waschkolonne weist jedoch typischerweise keinen Sumpfverdampfer auf.

Unter einer "Extraktionskolonne" wird im hier verwendeten Sprachgebrauch ein Flüssig-Flüssig-Extraktor verstanden, in dem eine Extraktion im Gegenstrom durchgeführt wird. Es können unterschiedliche Bauformen eingesetzt werden, denen jedoch ein gemeinsames Funktionsprinzip zu Grunde liegt. Insbesondere können Extraktionskolonnen senkrecht stehend ausgebildet sein, wobei oben kontinuierlich die spezifisch schwerere Flüssigkeit (im vorliegenden Fall das zu extrahierende Flüssigkeitsgemisch) und unten die spezifisch leichtere Flüssigkeit (im vorliegenden Fall das Extraktionsmittel) eingebracht wird. Für eine möglichst große Austauschfläche zwischen den Flüssigkeiten wird auch hier eine Feinverteilung vorgenommen. Insbesondere geeignete Vorrichtungen und Einbauten begünstigen eine intensive Durchmischung. Die spezifisch leichtere Flüssigkeit (mit Extrakt beladenes Lösungsmittel) wird am Kopf der Extraktionskolonne abgezogen.

Zur Auslegung und spezifischen Ausgestaltung von entsprechenden Kolonnen sei auf Lehrbücher verwiesen (siehe beispielsweise K. Sattler, "Thermische Trennverfahren: Grundlagen, Auslegung, Apparate", 3. Auflage, Wiley-VCH, Weinheim 2001).

Wie eingangs erwähnt, können in der ODH insbesondere bei Verwendung von MoVNbTeOx-Katalysatoren signifikante Mengen der jeweiligen Carbonsäuren der eingesetzten Paraffine als Nebenprodukte gebildet werden. Weitere Nebenprodukte sind unter anderem Kohlenmonoxid und Kohlendioxid. Ferner enthält ein einem ODH-Reaktor entnommenes Gasgemisch typischerweise Reaktions- und Prozesswasser, Restsauerstoff und Restethan. Ein solches Gasgemisch wird hier als "Prozessgas" der ODH bzw. "erstes Komponentengemisch" bezeichnet. Das Prozessgas enthält, wie erwähnt, ein oder mehrere Olefine als Hauptprodukt(e) sowie Nebenprodukte und nicht umgesetzte Edukte. Im Falle der ODH-E handelt es sich bei den Hauptprodukten um Ethylen und Essigsäure.

### Vorteile der Erfindung

Aus der US 2014/0249339 A1 ist bekannt, ein Prozessgas einer ODH einer Wasserwäsche zu unterwerfen, um dieses zu kühlen und wasserlösliche Komponenten aus diesem auszuwaschen (sogenannter Wasserquench). Bei der in einem derartigen Wasserquench anfallenden Flüssigkeit handelt es sich im Wesentlichen um eine wässrige Essigsäurelösung mit einem Gehalt von typischerweise 5-20 Gew.-%, beispielsweise ca. 11 Gew.-%, Essigsäure. Die Essigsäurelösung fällt dabei auf einer Temperatur von typischerweise 75 bis 100 °C, beispi elsweise ca. 90 °C, an. Sie wird nachfolgend auch als "zweites Komponentengemisch" bezeichnet. Es versteht sich, dass in einer bei einem entsprechenden Wasserquench anfallenden Essigsäurelösung auch weitere Komponenten, beispielsweise leichte Kohlenwasserstoffe, die zu einem gewissen Anteil ausgewaschen werden, enthalten sein können.

Zur Gewinnung der Essigsäure aus der Essigsäurelösung können grundsätzlich unterschiedliche Verfahren eingesetzt werden, beispielsweise konventionelle Destillationsverfahren, Lösungsmittelextraktionsverfahren und/oder Verfahren unter Verwendung geeigneter Entrainer. Insbesondere aus energetischen Gründen kommen im Stand der Technik bei stärker verdünnten Essigsäurelösungen typischerweise die zuletzt genannten Verfahren zum Einsatz.

Die vorliegende Erfindung umfasst nun ebenfalls einen entsprechenden Wasserquench, bei dem eine entsprechende wässrige Essigsäurelösung erhalten wird, und die Verwendung einer Flüssig-Flüssig-Extraktion der wässrigen Essigsäurelösung, beispielsweise unter Verwendung von Methyl-tert-Butylether (MTBE) oder Ethylacetat, gefolgt von einer Lösungsmitteldestillation. Die wässrige Essigsäurelösung wird dabei zunächst einer Extraktionskolonne zugeführt, die vorteilhafterweise unter atmosphärischen Bedingungen (Umgebungsdruck und Umgebungstemperatur) betrieben wird. Die Essigsäure wird in der Extraktionskolonne aus der wässrigen Essigsäurelösung unter Verwendung der genannten Lösungsmittel extrahiert. Das gewonnene Lösungsmittel-Essigsäuregemisch wird anschließend bei atmosphärischen Bedingungen zur Gewinnung von reiner Essigsäure in einer Destillationskolonne destilliert. Im Sumpf dieser Destillationskolonne fällt dabei im Wesentlichen reine Essigsäure an, am Kopf wird ein Komponentengemisch erhalten, das nachfolgend auch als "viertes" Komponentengemisch bezeichnet wird und das nicht in den Sumpf der Destillationskolonne übergegangene Essigsäure, geringe Mengen Wasser und ansonsten überwiegend Lösungsmittel enthält. Vor oder nach der Einspeisung in die Destillationskolonne wird das Lösungsmittel-Essigsäuregemisch erwärmt. Vorzugsweise erfolgt diese Erwärmung auf ein Temperaturniveau, das nahe der Verdampfungstemperatur des Lösungsmittel-Essigsäuregemischs liegt. Auf diese Weise lässt sich der Energiebedarf für die Destillation, der zu einem wesentlichen Anteil auf den Wärmebedarf eines in der Destillation eingesetzten Sumpfverdampfers zurückzuführen ist, im Rahmen der vorliegenden Erfindung reduzieren, wobei gleichzeitig, wie unten erläutert, andere Stoffströme effektiv gekühlt werden können.

Insgesamt schlägt die vorliegende Erfindung ein Verfahren zur Herstellung von Ethylen vor, bei dem Ethan in einem Reaktionseinsatz durch oxidative Dehydrierung (ODH, ODH-E) unter Anwesenheit von Sauerstoff unter Erhalt eines gasförmigen ersten Komponentengemischs, das zumindest Ethan, Ethylen, Essigsäure und Wasser enthält, teilweise katalytisch umgesetzt wird. Die vorliegende Erfindung kann damit grundsätzlich in einem bekannten Verfahren zur ODH-E zum Einsatz kommen. Zu weiteren Details sei auf die eingangs zitierte Fachliteratur verwiesen. Das in der ODH-E erhaltene erste Komponentengemisch kann einer Konditionierung, beispielsweise einer Vorkühlung, unterworfen werden, ehe es in dem erfindungsgemäßen Verfahren wie nachfolgend beschrieben behandelt wird. Bei dem ersten Komponentengemisch handelt es sich um ein Produktgas bzw. Prozessgas der ODH-E.

Ist hier davon die Rede, dass ein entsprechendes erstes Komponentengemisch Ethan, Ethylen, Essigsäure und Wasser enthält, schließt dies selbstverständlich nicht aus, dass in dem Komponentengemisch auch weitere Komponenten enthalten sein können, insbesondere Nebenprodukte der ODH-E oder in dem Reaktionseinsatz bereits enthaltene, jedoch in der ODH-E nicht umgesetzte Komponenten, die hier als inerte Komponenten bezeichnet werden. Eine inerte Komponente ist nicht notwendigerweise ein klassisches Inertgas sondern beispielsweise auch ggf. im Reaktionseinsatz vorhandenes Methan, das sich in der ODH-E nicht oder nur kaum umsetzt.

Die vorliegende Erfindung sieht vor, dass zumindest ein Teil des gasförmigen ersten Komponentengemischs unter Erhalt eines flüssigen zweiten Komponentengemischs, das Wasser und Essigsäure enthält, einer Wäsche mit einer Waschflüssigkeit unterworfen wird. Die Wäsche wird dabei insbesondere in einer Waschkolonne durchgeführt, wie sie zuvor erläutert wurde. Die Wäsche dient dabei nicht nur der Aufreinigung eines entsprechenden ersten Komponentengemischs sondern insbesondere auch zu dessen Abkühlung. Eine entsprechende Wäsche ist grundsätzlich aus dem Stand der Technik bekannt und wird auch als Wasserquench bezeichnet (siehe oben).

Ferner sieht die vorliegende Erfindung vor, dass ein erster Anteil des zweiten Komponentengemischs zur Bildung der Waschflüssigkeit verwendet wird, und dass ein zweiter Anteil des zweiten Komponentengemischs unter Erhalt eines flüssigen dritten Komponentengemischs, das ein organisches Lösungsmittel und Essigsäure enthält, einer Lösungsmittelextraktion unterworfen wird. Im Rahmen der vorliegenden Erfindung wird also ein Kreislauf gebildet, wobei die Waschflüssigkeit stets aus der Sumpfflüssigkeit der Waschkolonne gebildet wird. Vorteilhafterweise werden, wie auch nachfolgend noch erwähnt, der erste und der zweite Anteil des zweiten Komponentengemischs vor ihrer entsprechenden Verwendung abgekühlt. Hierin besteht ein wesentlicher Aspekt der vorliegenden Erfindung. Die Lösungsmittelextraktion erfolgt vorzugsweise unter Verwendung einer Extraktionskolonne, wie sie eingangs erläutert wurde.

Schließlich sieht die vorliegende Erfindung vor, dass zumindest ein Teil des flüssigen dritten Komponentengemischs erwärmt und unter Erhalt einer überwiegend oder ausschließlich Essigsäue enthaltenden Flüssigkeit einer Destillation unterworfen wird. Diese Destillation dient also zur Bereitstellung eines essigsäurereichen Produkts, insbesondere im Wesentlichen reiner Essigsäure (Eisessig). Durch den Einsatz der vorliegenden Erfindung lässt sich ein derartiges reines Produkt bereitstellen und damit die Synthesekapazität der ODH-E voll nutzen.

Im Rahmen der vorliegenden Erfindung wurde jedoch auch erkannt, dass insbesondere eine entsprechende Destillation vergleichsweise energieaufwendig ist, weil hier Heizmedien erforderlich sind, die nicht ohne zusätzlichen Energieaufwand bereitgestellt werden können. Insbesondere wird eine Destillation, wie sie im Rahmen der vorliegenden Erfindung zum Einsatz kommen kann, unter Verwendung eines mit Niederdruckdampf betriebenen Sumpfverdampfers durchgeführt. Entsprechender Niederdruckdampf muss aufwendig bereitgestellt werden und die Destillation erweist sich dadurch als der Hauptenergieverbraucher in einer entsprechenden Trennung. Hier setzt die Erfindung ebenfalls an.

Gemäß der vorliegenden Erfindung wird die Erwärmung des dritten Komponentengemischs oder dessen der Destillation unterworfenen Teils zumindest teilweise im Wärmetausch mit dem ersten Komponentengemisch und/oder mit dem ersten und/oder mit dem zweiten Anteil des zweiten Komponentengemischs durchgeführt. Mit anderen Worten wird das dritte Komponentengemisch bzw. dessen der Destillation unterworfenen Teil vor der Durchführung der Destillation (wie erwähnt ist aber auch eine Erwärmung mittels eines Zwischenaufkochers in der Destillation möglich) erwärmt oder sogar vor- bzw. vollverdampft. Dies ermöglicht es, die Destillation mit geringerem Energieaufwand durchzuführen, da geringere Mengen an Wärme und damit Niederdruckdampf im Sumpfverdampfer einer entsprechenden Destillationskolonne zur Verfügung gestellt werden müssen. Die Erwärmung kann dabei insbesondere auf einem Temperaturniveau erfolgen, welches unterhalb des Temperaturniveaus liegt, das für den Betrieb des Sumpfverdampfers erforderlich ist (40 bis 120 °C; 120 °C ist dabei der Siedepunkt der Ess igsäure und die mindestens benötigte Sumpftemperatur).

Die Erwärmung kann unter Verwendung eines oder mehrerer Wärmetauscher durchgeführt werden, durch die das dritte Komponentengemisch vor der Einspeisung in die Destillationskolonne geführt wird. In einer weiteren Ausgestaltung der Erfindung kann der Wärmeeintrag alternativ oder zusätzlich direkt in der Destillation mittels eines Zwischenaufkochers erfolgen, der ebenfalls entsprechend beheizt werden kann. Die Verwendung eines Zwischenaufkochers hat ebenso den Effekt der Energieeinsparung am Sumpf der Destillationskolonne. Auch in diesem Fall wird also zumindest ein Teil des flüssigen dritten Komponentengemischs erwärmt und unter Erhalt einer überwiegend oder ausschließlich Essigsäue enthaltenden Flüssigkeit einer Destillation unterworfen, wobei das dritte Komponentengemisch oder dessen der Destillation unterworfener Teil zumindest teilweise im Wärmetausch mit dem ersten Komponentengemisch und/oder mit dem ersten und/oder mit dem zweiten Anteil des zweiten Komponentengemischs durchgeführt wird. Lediglich der Wärmetausch erfolgt in diesem Fall an anderer Stelle.

Eine Erwärmung gemäß der soeben erläuterten Ausführungsform der vorliegenden Erfindung ist deswegen besonders günstig, weil sie durch Wärmeintegration erfolgen kann, wobei eine ohnehin erforderliche Abkühlung des ersten Komponentengemischs und/oder des ersten und/oder des zweiten Anteils des zweiten Komponentengemischs vorgenommen werden kann.

Wie bereits zuvor erwähnt, wird das zweite Komponentengemisch bzw. dessen erster bzw. zweiter Anteil vor seinem Einsatz als Waschflüssigkeit, aber auch vor seiner Einspeisung in die Lösungsmittelextraktion abgekühlt. Das zweite Komponentengemisch bzw. dessen erster bzw. zweiter Anteil liegt dabei auf einem zur Erwärmung des dritten Komponentengemischs besonders günstigen Temperaturniveau von insbesondere 80 bis 100 °C, be ispielsweise ca. 90 °C vor und der Umfang der Abkühlung auf ein Temperaturniveau von 25 bis 50 °C, beispielsweise ca. 40 °C, entspricht weitgehend dem Umfang der Erw ärmung des dritten Komponentengemischs. Auch das erste Komponentengemisch kann vor seiner Einspeisung in die Waschkolonne einer Abkühlung im Wärmetausch mit dem dritten Komponentengemisch unterworfen werden, so dass die in der Waschkolonne zu erbringende Kühlleistung, und damit die Erwärmung des zweiten Komponentengemischs, geringer ausfällt.

Im Rahmen der vorliegenden Erfindung weist das zweite Komponentengemisch, also die Sumpfflüssigkeit der Waschkolonne, insbesondere 5 bis 20 Gew.-%, beispielsweise ca. 11 Gew.-%, Essigsäure und im verbleibenden Anteil zu wenigstens 80 Gew.-% Wasser auf. Zu einem restlichen Anteil enthält das zweite Komponentengemisch weitere Nebenprodukte der ODH-E, wie sie zuvor erläutert wurden.

Wie bereits zuvor erwähnt, fällt das zweite Komponentengemisch, also die Sumpfflüssigkeit der Waschkolonne, insbesondere auf einem Temperaturniveau von 80 bis 100 °C an, das zweite Komponentengemisch wird also auf diesem Temperaturniveau gebildet. Der erste und/oder der zweite Anteil des zweiten Komponentengemischs werden, wie ebenfalls bereits erwähnt, auf ein Temperaturniveau von 25 bis 50 °C, insbesondere auf ein Temperaturniveau von beispielsweise ca. 40 °C abgekühlt.

Vorteilhafterweise wird die Lösungsmittelextraktion im Rahmen der vorliegenden Erfindung unter Verwendung zumindest eines Lösungsmittels mit einem Siedepunkt im Bereich von 40 bis 100 °C (bei Umgebungsdruck) durc hgeführt. Insgesamt lässt sich feststellen, dass die im Rahmen der vorliegenden Erfindung vorgeschlagenen Maßnahmen umso effektiver sind, je höher das Temperaturniveau des zweiten Komponentengemischs und je geringer der Druck des dritten Komponentengemisches ist. Ferner ist die vorliegende Erfindung umso vorteilhafter, je geringer die Verdampfungstemperatur des in der Lösungsmittelextraktion eingesetzten Lösungsmittels ist, weil auf diese Weise bewirkt wird, dass das Temperaturniveau, bei dem das dritte Komponentengemisch verdampft, entsprechend gering ist. Mit besonderem Vorteil kann dabei im Rahmen der vorliegenden Erfindung Methyl-tert-Butylether (MTBE) eingesetzt werden.

Diese Vorteile lassen sich insbesondere dann erzielen, wenn die Wäsche, ggf. die Lösungsmittelextraktion und die Destillation sowie die Anwärmung des dritten Komponentengemsiches auf einem Druckniveau durchgeführt werden, das im wesentlichen dem Atmosphärendruck entspricht, beispielsweise einem Druckniveau von 0,9 bis 1,2 bar (abs.), insbesondere ca. Atmosphärendruck. In der Lösungsmittelextraktion kann, beispielsweise alleine durch die Hydrostatik, das Druckniveau auch deutlich höher sein und insbesondere von der Kolonnenhöhe und den Dichten der eingesezten Fluide abhängen.

Wie auch eingangs bereits kurz angesprochen, wird im Rahmen der vorliegenden Erfindung in der Destillation ein gasförmiges viertes Komponentengemisch erhalten, das zumindest das oder die organischen Lösungsmittel aus der Lösungsmittelextraktion und geringe Mengen Wasser sowie Essigsäure enthält. Das Wasser kann vorteilhafterweise überwiegend in einem einfachen Flüssig-Flüssig-Abscheider abgeschieden werden. Die dabei erhaltene Wasserphase wird vorteilhafterweise zusammen mit der an Essigsäure abgereiccherten Wasserphase aus der Extraktion zumindest zum Teil einer Strippung zugeführt. In dieser Strippung werden das oder die organischen Lösungsmittel sowie andere leichte Nebenkomponenten zumindest zum Teil unter Verwendung eines Stripgases aus der Wasserphase ausgetrieben.

In besonderer Weise eignet sich im Rahmen der vorliegenden Erfindung dabei Niederdruckdampf auf einem Temperaturniveau von 120 bis 220 °C als Stripgas. Auch die Strippung stellt daher einen energieaufwendigen Trennschritt dar, insbesondere dann, wenn der Niederdruckdampf gezielt für das Verfahren bereitgestellt werden muss. Auch eine Erwärmung der an Essigsäure abgereiccherten Wasserphase aus der Extraktion und/oder der Wasserphase aus dem vierten Komponentengemisch kann daher von Vorteil sein, weil sich auf diese Weise die Flüchtigkeit des Lösungsmittels erhöht und die Strippung erleichtert wird. Diese Erwärmung erfolgt insbesondere auch durch Wärmetauscher mit dem ersten und/oder den zweiten Anteil des zweiten Komponentengemischs.

Auch eine Anlage zur Herstellung von Ethylen mit wenigstens einem Reaktor, der dafür eingerichtet ist, Ethan in einem Reaktionseinsatz durch oxidative Dehydrierung unter Anwesenheit von Sauerstoff unter Erhalt eines gasförmigen ersten Komponentengemischs, das zumindest Ethan, Ethylen, Essigsäure und Wasser enthält, teilweise katalytisch umzusetzen, ist Gegenstand der vorliegenden Erfindung. Die Anlage zeichnet sich durch wenigstens eine Waschsäule aus, die dafür eingerichtet ist, zum zumindest einen Teil des gasförmigen ersten Komponentengemischs unter Erhalt eines flüssigen zweiten Komponentengemischs, das Wasser und Essigsäure enthält, einer Wäsche mit einer ersten Waschflüssigkeit zu unterwerfen. Ferner sind Mittel bereitgestellt, die dafür eingerichtet sind, einen ersten Anteil des zweiten Komponentengemischs zur Bildung der Waschflüssigkeit zu verwenden, sowie wenigstens eine Extraktionskolonne, die dafür eingerichtet ist, einen zweiten Anteil des zweiten Komponentengemischs unter Erhalt eines flüssigen dritten Komponentengemischs, das zumindest ein organisches Lösungsmittel und Essigsäure enthält, einer Lösungsmittelextraktion zu unterwerfen. Schließlich sind Mittel vorgesehen, die dafür eingerichtet sind, zumindest einen Teil des flüssigen dritten Komponentengemischs zu erwärmen, sowie wenigstens eine Destillationskolonne, die dafür eingerichtet ist, das erwärmte flüssige dritte Komponentengemisch oder dessen erwärmten Anteil zumindest teilweise einer Destillation unter Erhalt einer überwiegend oder ausschließlich Essigsäure enthaltenden Flüssigkeit zu unterwerfen.

Zu Merkmalen und Vorteilen einer entsprechenden Anlage sei auf die zuvor unter Bezugnahme auf das erfindungsgemäß vorgeschlagene Verfahren erläuterten Merkmale und Vorteile ausdrücklich verwiesen. Vorteilhafterweise ist eine entsprechende Anlage dafür eingerichtet, ein entsprechendes Verfahren durchzuführen.

Die Erfindung wird nachfolgend unter Bezugnahme auf die beigefügte Zeichnung näher erläutert, welche eine bevorzugte Ausführungsform der Erfindung zeigt.

### Kurze Beschreibung der Zeichnung

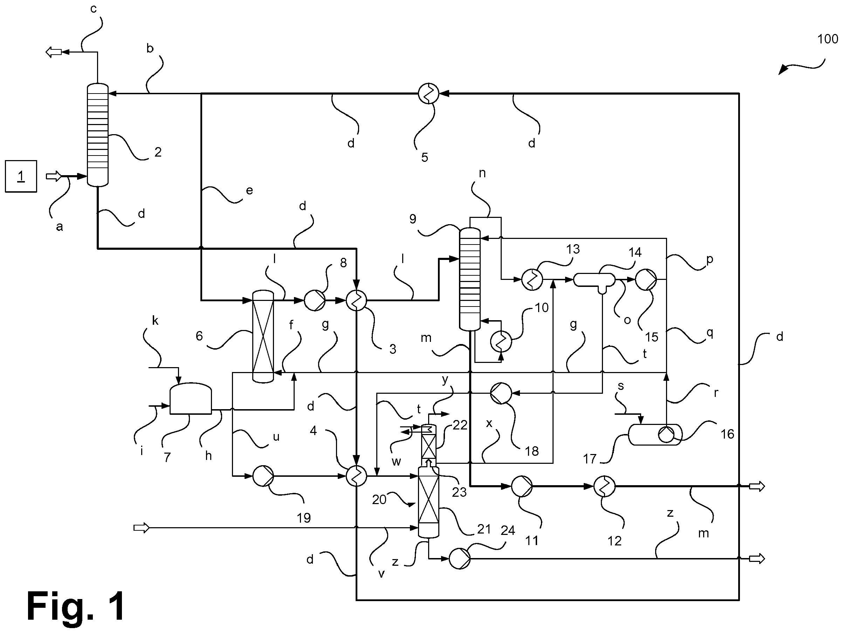

Figur 1 veranschaulicht ein Verfahren gemäß einer Ausführungsform der Erfindung.

### Ausführliche Beschreibung der Zeichnung

In Figur 1 ist ein Verfahren gemäß einer besonders bevorzugten Ausführungsform der vorliegenden Erfindung in Form eines schematischen Prozessflussdiagramms veranschaulicht und insgesamt mit 100 bezeichnet.

In dem Verfahren 100 wird ein Prozessgasstrom a aus einer hier nur stark schematisiert gezeigten ODH-E 1 einem unteren Bereich einer Waschkolonne 2 zugeführt, in deren oberem Bereich ein weiter unten beschriebener Stoffstrom b aufgegeben wird. Auf diese Weise wird zumindest der überwiegende Teil des in dem Prozessgasstrom a enthaltenen Wassers und der in dem Prozessgasstrom a enthaltenen Essigsäure im Sumpf der Waschkolonne 2 abgeschieden. Vom Kopf der Waschkolonne 2 kann auf diese Weise ein von zumindest einem überwiegenden Teil des enthaltenen Wassers und der enthaltenen Essigsäure befreiter Prozessgasstrom c erhalten werden.

Aus dem Sumpf der Waschkolonne 2 wird eine wässrige Essigsäurelösung in Form eines Stoffstroms d abgezogen. Wie bereits zuvor erwähnt, weist diese Essigsäurelösung beispielsweise einen Gehalt von ca. 11 Gew.-% Essigsäure und ein Temperaturniveau von beispielsweise ca. 90°C auf. Der Stoffstrom c wird in drei Wärmetauschern 3, 4 und 5 abgekühlt, von denen die Wärmetauscher 3 und 4 mit den nachfolgend noch erläuterten Stoffströmen l und u betrieben werden und der Wärmetauscher 5 beispielsweise mit Kühlwasser betrieben wird. Stromab des Wärmetauschers 5 wird ein Teil des Stoffstroms d in Form des bereits oben erwähnten Stoffstroms b abgezweigt. Auf diese Weise wird ein entsprechender Kreislauf geschaffen. Im oberen Bereich der Waschkolonne 2 wird, mit anderen Worten, ein Teil der aus dem Sumpf abgezogenen wässrigen Essigsäurelösung nach einer entsprechenden Kühlung aufgegeben.

Ein nach dem Abzweigen des Stoffstroms b verbleibender Reststrom, der besseren Anschaulichkeit halber nun mit e bezeichnet, wird auf einem Temperaturniveau von beispielsweise ca. 40°C in einen oberen Bereich ei ner Extraktionskolonne 6 eingespeist, die auf einem atmosphärischen Druckniveau und einem Temperaturniveau, dass der Umgebungstemperatur entspricht, betrieben wird. Die Extraktionskolonne 6 wird in einem unteren Bereich mit einem Lösungsmittelstrom f beschickt, der unter Verwendung eines wie unten erläutert gebildeten Lösungsmittelstroms g sowie eines aus einem Lösungsmitteltank 7 entnommenen Lösungsmittelstroms h gebildet wird. Der Lösungsmitteltank 7 seinerseits kann mit einem Lösungsmittelstrom i gespeist werden. Das Lösungsmittel kann aus dem Lösungsmitteltank 7 unter Verwendung eines Inertgasstroms k herausgedrückt werden. Bei dem Lösungsmittel kann es sich insbesondere um MTBE, bei dem Inertgas insbesondere um Stickstoff handeln.

Aus einem oberen Bereich der Extraktionskolonne 6 kann auf diese Weise ein im Wesentlichen Essigsäure und das verwendete Lösungsmittel enthaltender Stoffstrom l abgezogen werden. Dieser wird unter Verwendung einer Pumpe 8 durch den bereits erwähnten Wärmetauscher 3 geführt, in diesem erwärmt und dadurch vor- bzw. sogar vollverdampft, und in eine Destillationskolonne 9 eingespeist, die ebenfalls auf einem atmosphärischen Druckniveau betrieben wird. Wie bereits oben erwähnt, muss der Wärmetauscher 3 nicht gezwungendermaßen als separater Apparat für den Stoffstrom l vorliegen, sondern kann auch in Form eines Zwischenaufkochers in der Destillation 9 integriert werden.

Ein Sumpfverdampfer 10 der Destillationskolonne 9 kann insbesondere unter Verwendung von Niederdruckdampf betrieben werden. Durch die Erwärmung des Stoffstroms l kann, wie bereits erwähnt, im Rahmen der vorliegenden Erfindung durch einen geringeren Wärmebedarf in dem Sumpfverdampfer 10 eine Energieeinsparung erzielt werden. Gleichzeitig kann hierdurch eine gewünschte Abkühlung des Stoffstroms d bewirkt werden. Im Sumpf der Destillationskolonne 9 fällt im Wesentlichen reine Essigsäure (Eisessig) an, die in Form eines Stoffstroms m abgezogen, mittels einer Pumpe 11 durch einen mit Kühlwasser betriebenen Wärmetauscher 12 gepumpt und beispielsweise als Produkt an der Anlagengrenze abgegeben werden kann.

Das Kopfprodukt der Destillationskolonne 9 umfasst überwiegend das in der Extraktionskolonne 6 eingesetzte Lösungsmittel sowie geringe Mengen Wasser und Essigsäure. Dieses wird in Form eines Stoffstroms n abgezogen, durch einen mit Kühlwasser betriebenen Wärmetauscher 13 geführt und zusammen mit einem unten noch näher erläuterten Stoffstrom x in einen Abscheider 14 eingespeist. Aus diesem wird unter Verwendung einer Pumpe 15 ein überwiegend Lösungsmittel und geringe Mengen Essigsäure enthaltender Stoffstrom o abgezogen, welcher zu einem ersten Anteil in Form eines Stoffstroms p als Rücklauf auf die Destillationskolonne 9 zurückgeführt und zu einem zweiten Anteil in Form eines Stoffstroms q zur Bildung des bereits erwähnten Lösungsmittelstroms g verwendet wird. Der Stoffstrom q wird hierzu mit einem weiteren Lösungsmittelstrom r vereinigt, welcher mittels einer Pumpe 16 aus einem weiteren Lösungsmitteltank 17 gefördert wird. Der weitere Lösungsmitteltank 17 wird mit Lösungsmittel in Form eines Stoffstroms s gespeist. Ferner wird aus dem Abscheider 14 unter Verwendung einer Pumpe 18 ein überwiegend wässriger, jedoch auch noch Restanteile des eingesetzten Lösungsmittels enthaltender Stoffstrom t abgezogen, welcher wie unten näher erläutert verwendet wird.

Aus einem unteren Bereich der Extraktionskolonne 6 wird unter Verwendung einer Pumpe 19 ein überwiegend Wasser, jedoch auch noch Restanteile des eingesetzten Lösungsmittels enthaltender Stoffstrom u abgezogen, durch den bereits erwähnten Wärmetauscher 4 geführt, mit dem ebenfalls bereits erwähnten Stoffstrom t vereinigt und in eine Stripkolonne 20 eingespeist. In die Stripkolonne 20 wird Niederdruckdampf als Stripgas in Form eines Stoffstroms v eingespeist. Die Stripkolonne 20 weist einen unteren Kolonnenabschnitt 21 und einen oberen Kolonnenabschnitt 22 auf, wobei der untere Kolonnenabschnitt 21 und der obere Kolonnenabschnitt 22 durch einen Überlaufboden 23, beispielsweise einen Kaminhalsboden, voneinander getrennt sind. Der untere Kolonnenabschnitt 21 und der obere Kolonnenabschnitt 22 können je nach Bedarf unterschiedlich dimensioniert sein.

Der Kopf des oberen Kolonnenabschnitts 22 der Stripkolonne 20 kann beispielsweise unter Verwendung eines Kühlwasserstroms w gekühlt werden. Auf diese Weise bildet sich auf dem Überlaufboden 23 eine überwiegend das in dem Stoffströmen u und t enthaltene verbliebene Lösungsmittel enthaltende Flüssigkeit, welche in Form des bereits erwähnten Stoffstroms x abgezogen werden kann. Ein am Kopf des oberen Kolonnenabschnitts 22 der Stripkolonne 20 verbleibendes Kopfgas umfasst insbesondere leichte Kohlenwasserstoffe, die ebenfalls in der Quenchkolonne 2 in die flüssige Phase ausgewaschen wurden. Diese können beispielsweise abgefackelt oder anderweitig verwendet werden.

Aus dem Sumpf des unteren Kolonnenabschnitts 21 der Stripkolonne 20 wird ein überwiegend Wasser enthalten der Stoffstrom z mittels einer Pumpe 24 abgezogen und beispielsweise an der Anlagegrenze abgegeben.

## Patentansprüche

1. Verfahren (100) zur Herstellung von Ethylen, bei dem Ethan in einem Reaktionseinsatz durch oxidative Dehydrierung (1) unter Anwesenheit von Sauerstoff unter Erhalt eines gasförmigen ersten Komponentengemischs, das zumindest Ethan, Ethylen, Essigsäure und Wasser enthält, teilweise katalytisch umgesetzt wird, wobei zumindest ein Teil des gasförmigen ersten Komponentengemischs unter Erhalt eines flüssigen zweiten Komponentengemischs, das Wasser und Essigsäure enthält, einer Wäsche mit einer Waschflüssigkeit unterworfen wird, **dadurch gekennzeichnet, dass** ein erster Anteil des zweiten Komponentengemischs zur Bildung der Waschflüssigkeit verwendet wird, dass ein zweiter Anteil des zweiten Komponentengemischs unter Erhalt eines flüssigen dritten Komponentengemischs, das zumindest ein organisches Lösungsmittel und Essigsäure enthält, einer Lösungsmittelextraktion unterworfen wird, und dass zumindest ein Teil des flüssigen dritten Komponentengemischs erwärmt und unter Erhalt einer überwiegend oder ausschließlich Essigsäure enthaltenden Flüssigkeit einer Destillation unterworfen wird, wobei die Erwärmung des dritten Komponentengemischs oder dessen der Destillation unterworfenen Teils zumindest teilweise im Wärmetausch mit dem ersten Komponentengemisch und/oder mit dem ersten und/oder mit dem zweiten Anteil des zweiten Komponentengemischs durchgeführt wird.

2. Verfahren (100) nach Anspruch 1, bei dem die Erwärmung des dritten Komponentengemisch oder dessen der Destillation unterworfenen Teils auf ein Temperaturniveau von 40 bis 120 °C durchgeführt wird.

3. Verfahren (100) nach einem der vorstehenden Ansprüche, bei dem das zweite Komponentengemisch 5 bis 20 Gew.-% Essigsäure und im verbleibenden Anteil zu wenigstens 80 Gew.-% Wasser enthält.

4. Verfahren (100) nach einem der vorstehenden Ansprüche, bei dem das zweite Komponentengemisch auf einem Temperaturniveau von 80 bis 100 °C gebildet wird und der erste und/oder der zweite Anteil des zweiten Komponentengemischs auf ein Temperaturniveau von 25 bis 50 °C abgekühlt werden.

5. Verfahren (100) nach einem der vorstehenden Ansprüche, bei dem die Lösungsmittelextraktion unter Verwendung zumindest eines Lösungsmittels mit einem Siedepunkt im Bereich von 40 bis 100 °C (bei Umgebungsdruck) durchgeführt wird.

6. Verfahren (100) nach Anspruch 5, bei dem das zumindest eine Lösungsmittel Methyl-tert-Butylether umfasst.

7. Verfahren (100), bei dem zumindest die Wäsche und die Destillation auf einem Druckniveau von 0,9 bis 1,1 bar (abs.) durchgeführt werden.

8. Verfahren (100) nach einem der vorstehenden Ansprüche, bei dem in der Lösungsmittelextraktion ein weiteres Komponentengemisch gebildet wird, das überwiegend das Wasser aus dem zweiten Komponentengemisch und einen Anteil des oder der Lösungsmittel enthält, wobei das weitere Komponentengemisch im Wärmetausch mit dem ersten Komponentengemisch und/oder mit dem ersten und/oder mit dem zweiten Anteil des zweiten Komponentengemischs erwärmt wird.

9. Verfahren nach Anspruch 8, bei dem das weitere Komponentengemisch 1 bis 10 Masseprozent, insbesondere 1 bis 5 oder 2 bis 4 Masseprozent des oder der Lösungsmittel enthält.

10. Verfahren (100) nach Anspruch 8 oder 9, bei dem das weitere Komponentengemisch einer Strippung zugeführt wird.

11. Verfahren (100) nach einem der vorstehenden Ansprüche, bei dem die Destillation unter Beheizung mittels Niederdruckdampf durchgeführt wird.

12. Anlage zur Herstellung von Ethylen mit wenigstens einem Reaktor, der dafür eingerichtet ist, Ethan in einem Reaktionseinsatz durch oxidative Dehydrierung (1) unter Anwesenheit von Sauerstoff unter Erhalt eines gasförmigen ersten Komponentengemischs, das zumindest Ethan, Ethylen, Essigsäure und Wasser enthält, teilweise katalytisch umzusetzen, und mit wenigstens einer Waschsäule, die dafür eingerichtet ist, zumindest einen Teil des gasförmigen ersten Komponentengemischs unter Erhalt eines flüssigen zweiten Komponentengemischs, das Wasser und Essigsäure enthält, einer Wäsche mit einer Waschflüssigkeit zu unterwerfen, **gekennzeichnet durch** Mittel, die dafür eingerichtet sind, einen ersten Anteil des zweiten Komponentengemischs zur Bildung der Waschflüssigkeit zu verwenden, wenigstens einer Extraktionskolonne, die dafür eingerichtet ist, einen zweiten Anteil des zweiten Komponentengemischs unter Erhalt eines flüssigen dritten Komponentengemischs, das zumindest ein organisches Lösungsmittel und Essigsäure enthält, einer Lösungsmittelextraktion zu unterwerfen, Mittel, die dafür eingerichtet sind, zumindest einen Teil des flüssigen dritten Komponentengemischs zu erwärmen, wenigstens eine Destillationskolonne, die dafür eingerichtet ist, das erwärmte flüssige dritte Komponentengemisch oder dessen erwärmten Teil zumindest teilweise einer Destillation unter Erhalt einer überwiegend oder ausschließlich Essigsäure enthaltenden Flüssigkeit zu unterwerfen, und wenigstens einen Wärmetauscher, der dafür eingerichtet ist, die Erwärmung des dritten Komponentengemischs oder dessen der Destillation unterworfenen Teils zumindest teilweise im Wärmetausch mit dem ersten Komponentengemisch und/oder mit dem ersten und/oder mit dem zweiten Anteil des zweiten Komponentengemischs durchzuführen.

13. Anlage nach Anspruch 12, die zur Durchführung eines Verfahrens nach einem der Ansprüche 1 bis 11 eingerichtet ist.
